# EUROPEAN PATENT APPLICATION

(11) **EP 2 407 146 A1**
(43) Date of publication of application: **18.01.2012**
(21) Application number: 10007340.2
(22) Date of filing: 15.07.2010
(51) Int. Cl.: A61K 8/49, A61Q 11/00

(54) **Use of acesulfame K as a flavour modulator**

(71) Applicant: Nutrinova Nutrition Specialties & Food Ingredients GmbH, 65929 Frankfurt am Main (DE)
(72) Inventor: Rathjen, Susanne, 22337 Hamburg (DE)
(74) Representative: Lahrtz, Fritz

(57) **Abstract**

The present invention relates to the use of acesulfame K as a flavour modulator of a mint flavour in an oral hygiene product or in a dental product.

## Description

The present invention relates to the use of acesulfame K as a flavour modulator.

Today, many consumers, in particular consumers in the western world, try to avoid carbohydrates, in more particular many low-molecular weight sugars (mono- and disaccharides such as, e.g., glucose, fructose, sucrose, maltose, etc.). More than a billion people are overweight worldwide, from which at least 300 million are clinically obese. Overweight, in particular obesity, is a major risk of numerous chronic diseases such as type 2 diabetes, hypertension, stroke, cardiovascular diseases and certain types of cancer. Furthermore, carbohydrates, in particular many low-molecular weight sugars, bear a high glycemic index (GI). It is well-known that sugar uptake provokes systemic insulin release from the pancreas and, thereby, may cause a higher risk in developing type 2 diabetes. Moreover, many of the low-molecular weight sugars such as sucrose and degradation products of high-molecular weight carbohydrates (polysaccharides such as, e.g., starch, glycogen, amylose, amylopectin, etc.) may cause caries and cavities in the teeth.

On the other hand, saccharides and degradation products thereof, in particular low-molecular weight carbohydrates, may provoke a desirable sweet gustatory sensation. In this context, several products are not or hardly palatable when a sweet flavour.

Therefore, sweeteners are widely used as carbohydrate substitutes, in particular as sugar substitutes, in today's comestible goods and beverages.

One of the most common sweeteners is acesulfame K. Acesulfame K is a sweetener well-known to those skilled in the art. It was invented 1967 by the Hoechst AG (now Celanese/Nutrinova). Chemically it is the potassium salt of 6-methyl-1,2,3,-oxathiazin-4(3H)-one-2,2-dioxide

In contrast to some other sweeteners, acesulfame K is thermostable and may be, therefore, used in comestible goods and beverages that are heated during the production process or that are intended to be heated by the consumer, e.g. by cooking or baking. The acidity of acesulfame K is well-balanced (WO 04/084641 A1). Furthermore, acesulfame K may enhance the sensation of sweet compositions such as beverages containing a raspberry flavour (US 5,993,882).

Properties of acesulfame K are also described by Mitchell ("Sweeteners and Sugar alternatives in Food Technology'', Mitchell, H.L. (Editor), 2006, Blackwell Publishing Ltd., chapter 5.5.4. "Sweets and Chewing Gum", pages 77-78) and by Mayer and Kemper ("Acesulfame-K", Mayer, D.G. and Kemper F.H. (Editors), 1991, Marcel Dekker Inc., chapter "Oral Hygiene Products", page 223).

In other compositions, such as in oral hygiene products and dental products, additionally, a fresh flavour is desired.

The present invention relates to the use of acesulfame K as a flavour modulator of a mint flavour in an oral hygiene products or in a dental product.

As shown in the examples, acesulfame K serves as a flavour modulator, because it prolongs and enhances a mint flavour in toothpaste. Especially the mint taste was prolonged in the aftertaste, although the mint flavoured composition was disgorged and, thereby, removed from the oral cavity and was not swallowed.

In the context of the present invention, acesulfame K may be obtained from a commercial supplier, e.g. under the trade name "Sunett". Alternatively, acesulfame K may be obtained as a pure substance of a chemical supplier or may be synthesized. Exemplarily, acesulfame K may be obtained by the SO₃ method disclosed by European patent EP 0 155 634. Acesulfame K is commercially available from Nutrinova under the trade name "SUNETT". In the European Union it is classified and widely-used as food additive E950.

As used herein, the terms "acesulfame K", "acesulfame", "acesulfame potassium", "acesulfame-K", "Ace K", "Ace-K", "ACK", "Sunett'', "E950", and CAS-No. "55589-62-3" should be understood interchangeably. In the context of the present invention, acesulfame K may not be limited to the potassium salt, but may also relate to the free acid form as shown in EP 0 155 634 or to other salt forms such as acesulfame sodium, acesulfame calcium etc.

As used throughout the invention, the term "flavour modulator" may be understood in the broadest sense as a composition that enables the alteration of the gustatory sensation of a flavour.

The term "sweetener" may be understood interchangeably with "sweetening" or "sweetening agent". A sweetener may be an artificial sweetener (e.g. acesulfame K, saccharin, aspartame, cyclamate, sucralose, neotame, etc.) or a natural sweetener (e.g. steviol glycosides, lo han go, etc.).

The term "mint flavour" is known to the person skilled in the art. A mint flavour may be the gustatory sensation that is provoked in a subject, in particular a human, by exposing a leaf of a mint plant, an extract of a mint plant or menthol to the oral cavity, the nasopharynx and/or the tongue thereof. But it may be provoked by other substances as well. In the context of the present invention, the mint flavour may be provoked by a chemical composition. When administered to the mucosa of the oral cavity, the tongue and/or the nasopharynx, said chemical composition may provoke the mint flavour. The mouth may feel clean and smooth, subsequent to exposure of a mint flavour.

As used herein, the term "subject" refers to a person that is exposed to the respective flavour or a combination thereof or to a person that exposes himself or herself to the respective flavour or a combination thereof. Therefore, the term "subject" may be understood interchangeably with "person", "test person", "panelist", "proband", "consumer" or the like. In particular, the subject may be human.

In the context of the present invention, the terms "flavour", "taste", "sensation", gustatory sensation", "sensational characteristic", "taste characteristic", "gustatory characterisation", "aroma" and "gusto" as well as combinations thereof may be understood interchangeably.

In the context of the present invention, the acesulfame K and the mint flavour may be part of the same composition. However, it is also included in the present invention that they form part of two different compositions, which are administered simultaneously to the subject. In this context, simultaneously either means that both compositions are administered at the same time or that the effect of both compositions is present at the same time. This includes that one of the compositions is administered first, but that its effects are still present when the second composition is administered. In this context, either acesulfame K or the mint flavour may be administered first.

Throughout the invention, it is preferred that acesulfame K and the mint flavour form part of the same composition.

The composition comprising acesulfame K and/or a mint flavour may further contain other ingredients. In particular, these ingredients are ingredients typically used in an oral hygiene product or in a dental product. The composition may contain a liquid such as, e.g., water, ethanol and/or paraffin oil. Furthermore, it may contain one or more detergents, one or more foaming agents, one or more colouring agents, one or more vitamins, one or more salts, one or more humectants, one or more enzymes, one or more abrasives, one or more dental care agents, one or more preserving agents, one or more texturing agents, one or more gustatory substances, one or more fragrances and/or one or more pharmaceutical compositions.

As used in the context of the present invention, the term "aftertaste" may be understood in the broadest sense as a taste persisting in the mouth after the flavour that caused it is no longer present. In this context, flavour exposure may be terminated by physiologic or technical processes such as, e.g. swallowing, disgorging, rinsing, suction, cleaning and/or intensive dilution with a liquid or a solid.

Additionally, the composition comprising acesulfame K and/or the mint flavour may contain one or more additional sweeteners. In the context of oral hygiene products or dental products, the term "sweetener" may, preferably, refer to chemically synthesized sweet-tasting molecules that bear a comparably low glycemic index (GI) of < 0.5 of the GI of glucose. The gycemic index is well-known to those skilled in the art. Preferably, sweeteners may be High Intensity Sweeteners (HIS). The term "High Intensity Sweetener" refers to a molecule or a molecular composition that is sweeter than sucrose. Such HIS are well-known to those skilled in the art and may be, e.g. acesulfame K, alitame, aspartame, cyclamate, neohesperidine dihydrochalcone, lo han go, , neotame, saccharin, steviol glycosides, sucralose, thaumatin, etc. Preferably, aspartame K is combined with sucralose.

In a preferred embodiment, the mint flavour is prolonged by the use of acesulfame K as a flavour modulator. In the context of the present invention, the term "prolonged" means that the gustatory sensation lasts longer after the exposure of a mint flavour to the subject has been terminated.

In a further embodiment, the mint flavour is enhanced by the use of acesulfame K as a flavour modulator. In the context of the present invention, the term "enhanced" means that the gustatory sensation of the mint flavour is more intense.

In a preferred embodiment, the mint flavour comprises a monoterpene, in particular a monoterpene alcohol, more in particular wherein the mint flavour comprises menthol. Furthermore, the mint flavour may contain menthyl acetate, menthone or menthofurane or combinations thereof.

In a preferred embodiment, the mint flavour is an extract of a mint plant, in particular a peppermint (*Mentha* × *piperila, Mentha balsa mea*) plant, a field mint (*Mentha arvensis*) plant, a spearmint (*Mentha spicata*) plant, bergamot mint (*Mentha citrata*) plant, a water mint (*Mentha aqualica*) plant, a horse mint (*Mentha longifolia, Mentha sylvestris*) plant, a round-leaved mint (*Mentha rotundifolia*) plant, a garden mint (*Mentha sachalinensis*) plant, a *Mentha gracilis* plant, an apple mint (*Mentha suaveolens*) plant or hybrids thereof or mixtures thereof.

In an even more preferred embodiment, the flavour is mint oil obtained from field mint (*Mentha arvensis*) or peppermint oil obtained from peppermint (*Mentha piperita*). Typically, peppermint oil comprises 4.5 to 10 % menthylacetate, approximately 44 % menthol, 15 to 32 % menthone and menthofurane. Mint oil comprises approximately 3 to 17 % menthylacetate, approximately 42 % menthol, approximately 25 to 40 % menthone and no or nearly no menthofuran. There are four different stereoisomers of menthol, wherein in particular (-)-menthol bears the desired cooling properties and the typical mint aroma.

In a further preferred embodiment, the mint flavour is Optamint®. Optamint® is a commercially obtainable mint flavour additive offered by Symrise GmbH & Co. KG (Mühlenfeldstrasse 1, 37603 Holzminden, Germany).

In a preferred embodiment, the oral hygiene product or the dental product contains between 0.01 and 2 % (w/w), more preferably between 0.05 and 1 % (w/w), even more preferably between 0.1 and 1 % (w/w), even more preferably between 0.2 and 1 % (w/w), even more preferably between 0.25 and 0.75 % (w/w), even more preferably between 0.4 and 0.6 % (w/w) and most preferably 0.5 % (w/w) mint flavour.

In a preferred embodiment of the invention, the oral hygiene product or the dental product contains between 0.01 and 1 % (w/w), preferably between 0.04 and 0.75 % (w/w), more preferably between 0.08 and 0.5 % (w/w), even more preferably between 0.1 and 0.3 % (w/w) and most preferably 0.13 % (w/w) acesulfame K.

In a preferred embodiment, acesulfame K is used in an oral hygiene product.

Oral hygiene products are regularly used to prevent a disease of the oral cavity and/or the throat such as caries, oral cavities, gum diseases, periodontosis or to disinfect the mouth,. Alternatively, oral hygiene products may be used for cosmetic reasons such as to whiten or bleach the teeth or to prevent a bad breath or halitosis and/or to give a desired sensation and feel of the mouth. Furthermore, oral hygiene products may also be capable of cleaning and/or disinfection of a brace, implant or a denture in or out of the oral cavity.

In the context of the present invention, the term "oral hygiene product" may refer to any product that may be used in the context of dental care or dental hygiene which is either suitable for personal use or which may be used by an expert in the field of dentistry or odontology, such as a dentist, a dental surgeon or a dental assistant or a cosmetician. Preferably, oral hygiene products are suitable for personal use. The terms "oral hygiene product", "dental care product" and "dental hygiene product" may be understood interchangeably. Preferably, dental products as used herein are not intended to be swallowed.

In a more preferred embodiment of the present invention, the oral hygiene product is a toothpaste, a mouthwash, a mouth spray, a denture cleansing composition, a brace cleansing composition, a denture adhesive, a flavoured dental floss or a flavoured toothbrush.

A toothpaste as used herein, may be a dental care good that is used in order to clean the teeth and/or the oral cavity in order to remove plaque, prevent tooth and gum disease such as gingivitis, periodontal disease, caries, tooth cavities, calculus etc. The toothpaste may further contain typical ingredients of toothpastes. In particular the toothpaste may contain one or more liquids (e.g. water, ethanol, paraffin oil), one or more detergents, one or more foaming agents (e.g. sodium lauryl sulfate (SLS)/ sodium doceyl sulfate (SDS)), one or more colouring agents (e.g. TiO₂, food colouring), one or more vitamins, one or more salts (e.g. sodium, potassium, calcium, zinc salts), one or more humectants (e.g. sorbitol, glycerol, mannitol, propylenglycol, polydextrose), one or more enzymes, one or more abrasives, one or more dental care agents (e.g. fluorides, hydroxyapatites, calcium salts), one or more preserving agents (e.g. benzoic acid, methylparabene), one or more texturing agents (e.g. carboxymethyl cellulose (CMC), polyethylene glycol (PEG),), one or more emulsifiers , one or more bulking agents, one or more glacing agents, one or more separating agents, one or more antioxidants, one or more herbal and plant extracts (e.g. spearmint, peppermint, regular mint, Echinacea, eucalyptus, myrrh, Chamomile, Sage, Lavang, coriander, lemon), one or more homeopathic ingredients one or more gustatory substances, one or more fragrances and/or one or more pharmaceutical compositions.

A mouthwash may be used to prevent tooth and gum disease such as gingivitis, periodontal disease, caries, tooth cavities, calculus etc. A mouthwash may further contain typical ingredients of mouthwashes. In particular, a mouthwash may contain one or more liquids (e.g. water, ethanol, paraffin oil), one or more colours (e.g. a food colour), one or more vitamins, one or more salts (e.g. sodium, potassium, calcium, zinc salts), one or more enzymes, one or more dental care agents (e.g. fluorides, hydroxyapatites, calcium salts), one or more preserving agents (e.g. benzoic acid, methylparabene), one or more herbal and plant extracts (e.g. spearmint, peppermint, regular mint, Echinacea, eucalyptus, myrrh, Chamomile, Sage, Lavang, coriander, lemon), one or more homeopathic ingredients, one or more gustatory substances, one or more fragrances and/or one or more pharmaceutical compositions.

A mouth spray may be used to provoke a fresh sensation in the oral cavity and/or the nose and/or to give a fresh breathing. Furthermore, a mouth spray may have a therapeutic indication such as asthma or an allergic reaction. A mouth spray may further contain typical ingredients of mouth sprays.

A denture cleansing composition and a brace cleansing composition may be used to clean a denture and a brace, respectively, when taken out of the oral cavity. However, not cleaned within the oral cavity directly, it is preferred that aforementioned denture and brace have a fresh flavour when taken into the mouth again. The cleansing composition may be solid (e.g. a powder or a tablet), a liquid, a cream or a gel. The cleansing composition may further contain typical ingredients of a cleansing composition. In particular the cleansing composition may contain one or more liquids (e.g. water, ethanol, paraffin oil), one or more vitamins, one or more salts (e.g. sodium, potassium, calcium, zinc salts), one or more enzymes, one or more effervescent ingredients (e.g. carbonates and acids), one or more dental care agents (e.g. fluorides, hydroxyapatites, calcium salts), one or more preserving agents (e.g. benzoic acid, methylparabene), one or more herbal and plant extracts (e.g. spearmint, peppermint, regular mint, Echinacea, eucalyptus, myrrh, Chamomile, Sage, Lavang, coriander, lemon), one or more filling materials (e.g. sorbitol), one or more homeopathic ingredients, one or more gustatory substances, one or more fragrances and/or one or more pharmaceutical compositions.

A denture adhesive is used to attach the denture to the palate of the subject using said denture. The denture adhesive may preferably be a gel or a cream. It may further contain ingredient typically contained in a denture adhesive.

A dental floss and a toothbrush are used to remove plaque and residuals from the interspaces between the teeth and the teeth, respectively. A dental floss and a toothbrush, respectively, may be flavoured. It may further contain ingredients typically contained therein.

In another preferred embodiment, acesulfame K is used in a dental product. In the context of the present invention, the term "dental product" may refer to any product that may be used by an expert in the field of dentistry or odontology, such as a dentist, a dental surgeon or a dental assistant. Preferably, dental products as used herein are not intended to be swallowed.

Dental products may be used to support the experts in the field of dentistry or odontology to perform their work, such as, e.g., to make dental impressions, cleaning the teeth, a brace, a denture or to fit repair or remove teeth, a brace a denture and/or an implant.

In a more preferred embodiment, the dental product is a plastic material for dental impressions, a medicinal rinsing liquid, a denture, a brace, a denture cleaning device, a brace cleaning device or a dental prosthesis. A plastic material for dental impression may contain a matrix of an organic polymer material such as sodium alginate, polyether, silicones, condensation-cured silicones and addition-cured silicones or as polyvinyl siloxane. A medicinal rinsing liquid may be used to remove blood, saliva, pieces of teeth or plaque by washing, gargling or suction or to disinfect the oral cavity or certain teeth. A denture, a brace, a dental prosthesis, a denture cleaning device or a brace cleaning device may be flavoured by incorporation of a flavour in the plastic materials or by coating said materials with a mint flavour. A denture may be coated with a denture cleansing composition. Likewise, a brace may be coated with a brace cleansing composition.

In a further preferred embodiment of the invention, the dental product is a pharmaceutical composition. As used herein, a pharmaceutical composition may be understood in the broadest sense. Preferably, the pharmaceutical composition is a composition used by an expert in the field of dentistry or odontology, such as a dentist, a dental surgeon or a dental assistant. Preferably, it may be used to anoint one or more teeth or the gingival, to disinfect the oral cavity or regions of the oral cavity or to anesthetize a region in the oral cavity.

In a more preferred embodiment, the pharmaceutical composition is a tablet, a pill, a chewable tablet, a capsule, a chewable capsule, a syrup, a spray, a gel, a juice, a liquid or a paste. In this context, it may be understood that aforementioned pharmaceutical compositions may contain a large number of further ingredients typically contained in a pharmaceutical composition such as pharmaceutically active ingredients, colouring agents, excipients, adjuvants, filling material etc.

Furthermore, the invention refers to a method for modulating a mint flavour in an oral hygiene product or a dental product, wherein said modulation is conferred by the acesulfame K

All embodiments defined above with respect to the claimed use of the invention also apply to this method of the invention.

### Cited References

EP 0 155 634
US 5,993,882
WO 04/084641
"Sweeteners and Sugar alternatives in Food Technology", Mitchell, H.L. (Editor), 2006, Blackwell Publishing Ltd., chapter 5.5.4. "Sweets and Chewing Gum", pages 77-78 "Acesulfame-K", Mayer, D.G. and Kemper F.H. (Editors), 1991, Marcel Dekker Inc., chapter "Oral Hygiene Products", page 223

### Examples

The following examples *inter alia* demonstrate that acesulfame K may modulate a mint flavour. In particular, acesulfame K may prolong and/or enhance a mint flavour. The examples may exemplify the invention but are not intended to limit the scope thereof.

### Example 1

In the first example two different toothpaste compositions were compared with each other. Said toothpastes comprised two different sweeteners, namely, saccharin Na and acesulfame K and the mentholic flavour Optamint®. Basis of the analysis was a sensory analysis performed by a sensory panel of three experts accredited with DIN EN ISO/IEC 17025. Herein, the term "DIN" refers to "Deutsches Institut für Normung". The sensory test was performed according to the requirements of DIN 10964.

The toothpaste compositions are described in the following:

**Table 1: Toothpaste composition 1 (EVONUK 25855)**

| Raw materials | % (w/w) |
|---|---|
| SIDENT® 9 | 14.00 |
| SIDENT®22 S | 8.00 |
| sorbitol 70% | 50.00 |
| water | 23.38 |
| sodium lauryl sulphate | 1.50 |
| NaCMC (7MXF) | 1.20 |
| paraffin oil | 0.50 |
| **Optamint® #797966** | **0.50** |
| TiO₂ | 0.40 |
| sodium fluoride | 0.22 |
| methylparabene Na salt | 0.20 |
| **saccharin Na** | **0.10** |

**Table 2: Toothpaste composition 2 (EVONUK 25856)**

| Raw materials | % (w/w) |
|---|---|
| SIDENT® 9 | 14.00 |
| SIDENT®22 S | 8.00 |
| sorbitol 70% | 50.00 |
| water | 23.55 |
| sodium lauryl sulphate | 1.50 |
| NaCMC (7MXF) | 1.20 |
| paraffin oil | 0.50 |
| **Optamint® #797966** | **0.30** |
| TiO₂ | 0.40 |
| sodium fluoride | 0.22 |
| methylparabene Na salt | 0.20 |
| **acesulfame K** | **0.13** |

The experts brushed their teeth with the respective toothpastes by means of manual toothbrushes for approximately 1 to 2 min. Subsequently, the oral cavity was rinsed thoroughly with water for approximately 10-20 seconds.

The gustatory sensations while brushing the teeth and the sensations of the aftertaste were collected and listed. Between testing the different samples, the experts paused and regenerated for at least 10 min and until the aftertaste was entirely vanished.

The result of example 1 is depicted in table 1. Notably, the fresh taste lingers longer, when the toothpaste comprises acesulfame K.

**Table 3: Sensory results of example 1**

| Toothpaste | sweetener | Optamint® | sensation |
|---|---|---|---|
| EVONUK 25855 | 0.1 % (w/w) saccharin Na | 0.5 % (w/w) | slightly sweet; intensively mentholic; slightly hot and burning; slightly blunt sensation as mentholic taste prevails |
| EVONUK 25856 | 0.13 % (w/w) acesulfame K | 0.3 % (w/w) | slightly sweet at the beginning, later slightly mentholic; lingers after rinsing the mouth and in the aftertaste; slightly blunt sensation (abrasives clearly sensible); no bitter taste sensible |

The experts suggested using the same concentration of acesulfame K, but a higher concentration of Optamint®.

### Example 2

In the second example four different toothpastes were compared with each other. Said toothpastes comprised two different sweeteners, namely, saccharin Na and acesulfame K and the mentholic flavour Optamint® in various concentrations. Basis of the analysis was a sensory analysis performed by a sensory panel of three experts accredited with DIN EN ISO/IEC 17025. The sensory test was performed according to the requirements of DIN 10964.

The toothpaste compositions are described in the following:

**Table 4: Toothpaste composition 1 (EVONUK 25855)**

| Raw materials | % (w/w) |
|---|---|
| SIDENT®9 | 14.00 |
| SIDENT®22 S | 8.00 |
| sorbitol 70% | 50.00 |
| water | 23.38 |
| sodium lauryl sulphate | 1.50 |
| NaCMC (7MXF) | 1.20 |
| paraffin oil | 0.50 |
| **Optamint® #797966** | **0.50** |
| TiO₂ | 0.40 |
| sodium fluoride | 0.22 |
| methylparabene Na salt | 0.20 |
| **saccharin Na** | **0.10** |

**Table 5: Toothpaste composition 2 (EVONUK 25856)**

| Raw materials | % (w/w) |
|---|---|
| SIDENT® 9 | 14.00 |
| SIDENT®22 S | 8.00 |
| sorbitol 70% | 50.00 |
| water | 23.55 |
| sodium lauryl sulphate | 1.50 |
| NaCMC (7MXF) | 1.20 |
| paraffin oil | 0.50 |
| **Optamint® #797966** | **0.30** |
| TiO₂ | 0.40 |
| sodium fluoride | 0.22 |
| methylparabene Na salt | 0.20 |
| **acesulfame K** | **0.13** |

**Table 6: Toothpaste composition 3 (EVONUK 25859)**

| Raw materials | % (w/w) |
|---|---|
| SIDENT® 9 | 14.00 |
| SIDENT®22 S | 8.00 |
| sorbitol 70% | 50.00 |
| water | 23.35 |
| sodium lauryl sulphate | 1.50 |
| NaCMC (7MXF) | 1.20 |
| paraffin oil | 0.50 |
| **Optamint® #797966** | **0.50** |
| TiO₂ | 0.40 |
| sodium fluoride | 0.22 |
| methylparabene Na salt | 0.20 |
| **acesulfame K** | **0.13** |

**Table 7: Toothpaste composition 4 (EVONUK 25860)**

| Raw materials | % (w/w) |
|---|---|
| SIDENT® 9 | 14.00 |
| SIDENT®22 S | 8.00 |
| sorbitol 70% | 50.00 |
| water | 23,15 |
| sodium lauryl sulphate | 1.50 |
| NaCMC (7MXF) | 1.20 |
| paraffin oil | 0.50 |
| **Optamint® #797966** | **0.70** |
| TiO₂ | 0.40 |
| sodium fluoride | 0.22 |
| methylparabene Na salt | 0.20 |
| **acesulfame K** | **0.13** |

### The test procedure was as above.

The result of example 2 is depicted in table 2.

**Table 8: Sensory results of Example 2**

| toothpaste | sweetener | Optamint® | Sensation |
|---|---|---|---|
| EVONUK 25855 | 0.1 % (w/w) saccharin Na | 0.5 % (w/w) | slightly sweet; intensively mentholic; slightly hot and burning; fresh in taste, declines rapidly, disappeared after rinsing the mouth; slightly blunt sensation |
| EVONUK 25856 | 0.13 % (w/w) acesulfame K | 0.3 % (w/w) | slightly sweet at the beginning, later slightly mentholic; lingers after rinsing the mouth and in the aftertaste |
| EVONUK 25859 | 0.13 % (w/w) acesulfame K | 0.5 % (w/w) | Slightly sweet; intensively mentholic; fresh; lingers after rinsing the mouth and in the aftertaste; slightly blunt sensation |
| EVONUK 25860 | 0.13 % (w/w) acesulfame K | 0.7 % (w/w) | Slightly sweet; intensively mentholic; very hot and burning; fresh; lingers after rinsing the mouth and in the aftertaste; slightly blunt sensation |

The toothpaste composition EVONUK 25859 comprising 0.13 % (w/w) acesulfame K and 0.5 % (w/w) Optamint® was described as the most desired composition. The sensation while brushing the teeth is comparable to the sensation provoked by EVONUK 25855. However, the desired fresh taste lingers longer.

## Claims

1. Use of acesulfame K as a flavour modulator of a mint flavour in an oral hygiene product or in a dental product.

2. The use according to claim 1, wherein the mint flavour is prolonged.

3. The use according to any of claims 1 or 2, wherein the mint flavour is enhanced.

4. The use according to any of claims 1 to 3, wherein the mint flavour comprises a monoterpene, in particular wherein the mint flavour comprises menthol.

5. The use according to any of claims 1 to 5, wherein the mint flavour is an extract of a mint plant, in particular a peppermint (*Mentha* × *piperita, Mentha balsamea*) plant, a field mint (*Mentha arvensis*) plant, a spearmint (*Mentha spicata*) plant, bergamot mint (*Mentha citrata*) plant, a water mint (*Mentha aquatica*) plant, a hourse mint (*Mentha longifolia, Mentha sylvestris*) plant, a round-leaved mint (*Mentha rotundifolia*) plant, a garden mint (*Mentha sachalinensis*) plant, a *Mentha gracilis* plant, an apple mint (*Mentha suaveolens*) plant or hybrids thereof or mixtures thereof.

6. The use according to any of claims 1 to 5, wherein the mint flavour is Optamint®.

7. The use according of any of claims 1 to 6, wherein the oral hygiene product or the dental product contains between 0.01 and 1 % (w/w), more preferably between 0.05 and 1 % (w/w), even more preferably between 0.1 and 1 % (w/w), even more preferably between 0.2 and 1 % (w/w), even more preferably between 0.25 and 0.75 % (w/w), even more preferably between 0.4 and 0.6 % (w/w) and most preferably 0.5 % (w/w) mint flavour.

8. The use according of any of claims 1 to 7, wherein the oral hygiene product or the dental product contains between 0.01 and 1 % (w/w), preferably between 0.04 and 0.75 % (w/w), more preferably between 0.08 and 0.5 % (w/w), even more preferably between 0.1 and 0.3 % (w/w) and most preferably 0.13 % (w/w) acesulfame K.

9. The use according to any of claims 1 to 8, wherein acesulfame K is used in an oral hygiene product.

10. The use according to claim 9, wherein the oral hygiene product is a toothpaste, a mouthwash, a mouth spray, a denture cleansing composition, a brace cleansing composition, a denture adhesive, a flavoured dental floss or a flavoured toothbrush.

11. The use according to any of claims 1 to 8, wherein acesulfame K is used in a dental product.

12. The use according to claim 11, wherein the dental product is a plastic material for dental impressions, a medicinal rinsing liquid, a denture, a brace, a denture cleaning device, a brace cleaning device or a dental prosthesis.

13. The use according to claim 11, wherein the dental product is a pharmaceutical composition.

14. The use according to claim 13, wherein the pharmaceutical composition is a tablet, a pill, a chewable tablet, a capsule, a chewable capsule, a syrup, a spray, a gel, a juice, a liquid or a paste.
